# EUROPEAN PATENT SPECIFICATION

(11) **EP 0 654 042 B1**
(45) Date of publication and mention of the grant of the patent: **07.10.1998**
(21) Application number: 93911125.8
(22) Date of filing: 12.05.1993
(51) Int. Cl.: C07K 14/575, A61K 38/22, G01N 33/50

(54) **AMYLIN MUTEINS**
AMYLIN MUTEINE
MUTEINES D'AMYLINE

(30) Priority: 06.08.1992 US 926783
(43) Date of publication of application: 24.05.1995
(73) Proprietor: PFIZER INC., New York, N.Y. 10017 (US)
(72) Inventor: ANDREWS, Glenn, Colton, Waterford, CT 06385 (US); KREUTTER, David, Kevin, Madison, CT 06443 (US)
(74) Representative: Hayles, James Richard
(86) International application number: US9304314
(87) International publication number: WO9403491

(56) References cited:
- EP-A- 0 309 100
- WO-A-89/06135

### SEQUENCE LISTING

(1) GENERAL INFORMATION:
   (i) APPLICANT:
      (A) NAME: Pfizer Inc.
      (B) STREET: 235 East 42nd Street
      (C) CITY: New York
      (D) STATE: New York
      (E) COUNTRY: U.S.A.
      (F) POSTAL CODE (ZIP): 10017-5755
      (G) TELEPHONE: 212 573-2841
      (H) TELEFAX: 212 573-1939
   (ii) TITLE OF INVENTION: Amylin Muteins
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 37 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 37 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 2:
   Lys Cys Asn Thr Ala Thr Cys Ala Thr Gln Arg Leu Ala Asn Phe Leu
   1               5                   10                  15
   Val Arg Ser Ser Asn Asn Leu Gly Pro Val Leu Pro Pro Thr Asn Val
               20                  25                  30
   Gly Ser Asn Thr Tyr
           35
(2) INFORMATION FOR SEQ ID NO: 3:
   (i) SEQUENCE CHARACTERISTICS:
      (A) LENGTH: 37 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION:15
      (D) OTHER INFORMATION:/product= "OTHER" /note= "Tyr, mono-iodinated Tyr or bis-iodinated Tyr"
   (xi) SEQUENCE DESCRIPTION: SEQ ID NO: 3:
   Lys Cys Asn Thr Ala Thr Cys Ala Thr Gln Arg Leu Ala Asn Xaa Leu
   1               5                   10                  15
   Val His Ser Ser Asn Asn Phe Gly Ala Ile Leu Ser Ser Thr Asn Val
               20                  25                  30
   Gly Ser Asn Thr Phe
           35
(2) INFORMATION FOR SEQ ID NO: 4:
   (i) SEQUENCE CHARACTERISTICS:
      (A) LENGTH: 37 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION:15
      (D) OTHER INFORMATION:/product= "OTHER" /note= "Tyr, mono-iodinated Tyr or bis-iodinated Tyr"
   (xi) SEQUENCE DESCRIPTION: SEQ ID NO: 4:
   Lys Cys Asn Thr Ala Thr Cys Ala Thr Gln Arg Leu Ala Asn Xaa Leu
   1               5                   10                  15
   Val Arg Ser Ser Asn Asn Leu Gly Pro Val Leu Pro Pro Thr Asn Val
               20                  25                  30
   Gly Ser Asn Thr Phe
           35

## Description

### Field of the Invention

This invention relates to muteins of amylin and derivatives thereof having insulin action suppressing activity as well as the pharmaceutically-acceptable salts thereof. More specifically, this invention relates to muteins of human amylin, muteins of rat amylin and derivatives thereof having insulin action suppressing activity as well as the pharmaceutically-acceptable salts thereof. The muteins and derivatives of this invention are useful for suppressing insulin action in muscle or muscle cells and for suppressing insulin action in a mammal in need thereof. Thus, this invention further relates to a method of suppressing insulin action in a mammal in need thereof and methods for suppressing insulin action in muscle or muscle cells. Still further, this invention relates to a method for assaying the ability of a compound to inhibit the suppression of insulin action in muscle or muscle cells caused by amylin. Still further yet, this invention relates to pharmaceutical compositions comprising the above described muteins, derivatives or salts.

### Background Art

Human amylin is a 37 amino acid polypeptide having a primary amino acid sequence of

Rat amylin is a 37 amino acid polypeptide having a primary amino acid sequence of

Amylin contains a disulfide bond between the Cys residues at positions 2 and 7. It is a secretory product of pancreatic β cells. In the type II diabetic, there are extensive deposits of amylin derived amyloid in the pancreas and it has been postulated that the amyloid contributes to the pathology associated with the disease. Westermark, P., et al., Biochem. Biophys. Res. Commun. 140:827-831 (1986); Clark, A., et al., Lancet ii:231-234 (1987); Westermark, P., et al., Diabetologia 30:887-892 (1987); Cooper, G. J. S., et al., Proc. Natl. Acad. Sci. USA 84:8528-8532 (1987); Westermark, P., et al., Proc. Natl. Acad. Sci. USA 84:3881-3885 (1987); Westermark, P., et al., Am. J. Pathol. 127:414-417 (1987); Clark, A., et al., Diabetologia 33:285-289 (1990).

In isolated soleus muscle, amylin suppresses insulin-stimulated glycogen synthesis. Leighton, B., et al., Nature 335:632-635 (1988); Cooper, G. J. S., et al., Proc. Natl. Acad. Sci. USA 85:7763-7766 (1988). The effect of amylin on insulin action in skeletal muscle in vivo has been reported to be the inhibition of glycogenesis which is the major effect in isolated soleus muscle described above.

The use of amylin and calcitonin gene-related peptide to reduce insulin stimulated glycolysis in muscle or muscle cells and a method for assaying for antagonists thereof are disclosed in EP-A-0 503 827.

Certain amylin antagonists and the use thereof are described in WO89/06135 (PCT/US89/00049), published July 13, 1989.

EP-A-309100 discloses amylin, amylin-NH₂, CGRPs and conservative variants thereof for use in the treatment of diabetes mellitus or hypoglycaemia.

### Disclosure of the Invention

This invention relates to polypeptides comprising the primary sequence

H₂N-R-CONH₂

wherein R is an amino acid sequence selected from the group consisting of and or a derivative thereof having insulin action suppressing activity, and the pharmaceutically acceptable salts thereof wherein Xaa is Tyr, mono-iodinated Tyr or bis-iodinated Tyr.

Preferred polypeptides of this invention are those of SEQUENCE ID NO:3, above. Still more preferred polypeptides of this invention are those of SEQUENCE ID NO:3 wherein there is a disulfide bond between the Cys residue at position 2 and the Cys residue at position 7.

This invention also relates to a method for suppressing insulin action in muscle or muscle cells in the presence of an insulin action suppressing amount of a polypeptide according to this invention.

Further, this invention relates to a method for assaying the ability of a compound to inhibit the suppression of insulin action in muscle or muscle cells caused by amylin which method comprises:
(a) incubating muscle or muscle cells in the presence of insulin, a polypeptide or derivative thereof according to this invention, the compound and glucose;
(b) measuring the amount of glycogen produced by the incubated muscle or muscle cells of step (a); and
(c) comparing the amount measured in step (b) to the amount measured according to step (b) when muscle or muscle cells are incubated in the presence of insulin, the polypeptide or derivative thereof and glucose.

In the method described immediately above, the glucose used is preferably radiolabeled and the measurement of the amount of glycogen is preferably the measurement of the amount of radiolabeled glycogen.

Further, this invention relates to pharmaceutical compositions comprising a polypeptide according to this invention and a pharmaceutically-acceptable diluent or carrier.

Further still, this invention relates to a method for suppressing insulin action in a mammal in need thereof which method comprises administering to said mammal an effective amount of a polypeptide according to this invention.

### Detailed Description

The term "derivative" as used throughout this Specification and the appendant claims, includes, but is not limited to, polypeptides comprising an amino acid sequence of sufficient homology to SEQUENCE ID NO:3 or SEQUENCE ID NO:4, as well as variants thereof having inconsequential amino acid substitutions, all of which possess insulin action suppressing activity.

The phrase "insulin action suppressing activity", as used throughout this Specification and the appendant claims, includes, but is not limited to, the ability of a polypeptide of this invention to suppress the effect which insulin has upon muscle or muscle cells. By way of example and not of limitation, the polypeptides of this invention suppress insulin-stimulated glycogen synthesis in muscle or muscle cells.

For the purposes of this Specification and the appendant claims, all amino acids herein are L-amino acids with the obvious exception of Gly which is not so classified due to the presence of only hydrogen at the α-carbon in addition to the carboxyl and amino groups thereof.

In the expression "H₂N-R-CONH₂", as used throughout this Specification and the appendant claims, the H₂N- portion thereof refers to the N-terminal amino group of the N-terminal amino acid of sequence R and the -CONH₂ portion refers to the amide form of the C-terminal carboxyl group of the C-terminal amino acid of sequence R.

The term "muscle" as used throughout this Specification and the appendant claims includes intact muscle in vivo as well as various ex vivo preparations of muscle such as strips thereof.

The phrase "muscle cells" as used throughout this Specification and the appendant claims includes all muscle cells whether present in intact muscle in vivo, various ex vivo preparations of muscle or in in vitro cultures of muscle cells per se.

While the polypeptides of this invention are shown in the Specification and appendant claims in the form of primary amino acid sequences, it is to be understood that included within the scope of this invention are such polypeptides wherein there is a disulfide bond between Cys residue 2 and Cys residue 7.

The polypeptides of this invention are prepared by various methods well known to those skilled in the art. For example, the polypeptides can be synthesized using automated peptide synthesizers such as Applied Biosystems (ABI) 430A solid phase peptide synthesizer with standard tBOC chemistry protocols as provided by ABI (Version 1.4 for NMP/HOBt chemistry). Such protocols use hydoxybenzotriazole active ester coupling and acetic anhydride capping of unreacted free amino termini after each coupling. Resin samples are taken automatically at the end of each coupling and quantitative ninhydrin assay is used to monitor coupling efficiencies.

It has been found that amylin which has been mono- or bis-iodinated at the Tyr residue of position 37 will not suppress insulin action. Further, it also has been found that a Bolton-Hunter derivative of amylin of the formula 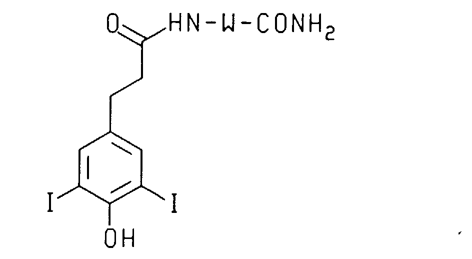 wherein W is SEQUENCE ID NO:1 will not suppress insulin action. In contrast, the polypeptides of this invention, which are muteins of SEQUENCE ID NO:1 and SEQUENCE ID NO:2, suppress insulin action and, inter alia, provide iodinated forms of the polypeptide which are useful in a variety of assays for the study of amylin action and antagonists thereof.

The method according to this invention wherein the ability of a compound to inhibit the suppression of insulin action in muscle or muscle cells caused by amylin is performed as described hereinbelow. The method comprises incubating muscle or muscle cells in the presence of insulin, a polypeptide or derivative thereof according to this invention, a compound to be assayed and glucose; measuring the amount of glycogen produced by the incubated muscle or muscle cells; and comparing the amount so measured to the amount of glycogen produced when muscle or muscle cells are incubated in the presence of insulin, the polypeptide or derivative thereof and glucose.

In the above described method, the amount of glycogen produced by the incubated muscle or muscle cells can be measured as follows. Weighed muscle or muscle cells are dissolved in 3 ml of 30% KOH by heating in a boiling water bath for about 20 - 30 minutes. After cooling, the glycogen is precipitated with 1.2 volumes of 95% ethanol. Then, the sample is heated to boiling and, after cooling, is centrifuged at 3000 rpm to pellet the glycogen. The pellet is redissolved in water and reprecipitated with ethanol. Then, the glycogen is hydrolyzed by adding 6 ml of 0.6 N HCI, covering the tube with a glass marble and heating in a boiling water bath for about 2 - 2.5 hours. The solution is cooled and neutralized. Then, the resultant liberated glucose is determined using an automated glucose analyzer.

It is preferable, however, to practice the above method by employing radiolabeled glucose and measuring the amount of radiolabled glycogen produced, all as described hereinbelow.

For the purposes of the above described method, it is preferable to employ rat soleus muscle which has been dissected and stripped into pieces weighing about 25-35 mg according to the procedure described by Crettaz, M., et al., Biochem. J. 186:525-534 (1980). The muscle pieces are placed in appropriate vessels containing gassed (95% O₂/5% CO₂) Krebs-Ringer bicarbonate buffer containing 1.2 mM calcium. An appropriate vessel for such muscle pieces is a 50 ml Erlenmeyer flask containing 4 ml of the gassed Krebs-Ringer bicarbonate buffer containing 1.2 mM calcium as described above. While not essential to the practice of the method of this invention, it is preferable to incubate the muscle pieces in said buffer at 37°C (shaking bath water) for 15 minutes under an atmosphere of 95% O₂/5%CO₂ followed by incubation for 15 minutes at 37°C (shaking water bath) in said buffer which additionally contains insulin, a polypeptide or derivative thereof according to this invention and a compound to be assayed prior to incubation at 37°C in the presence of insulin, a polypeptide or derivative thereof according to this invention, radiolabeled glucose and a compound to be assayed. Each of such subsequent incubations are preferably carried out in 4 ml of the respective buffer in 50 ml Erlenmeyer flasks.

Incubation of the muscle pieces in said buffer containing insulin, a polypeptide or derivative thereof according to this invention, radiolabeled glucose and a compound to be assayed is conducted at 37°C (shaking water bath) where the buffer is gassed (95% O₂/5% CO₂) for 60 minutes, also at 37°C (shaking water bath) with stoppered vessels. For the purpose of the above method, it is preferable to use insulin at a level of about 10⁻¹⁰ M to about 10⁻⁷ M. It also is preferable to use a polypeptide or derivative thereof according to this invention at a level of about 10⁻⁸ M to about 10⁻⁷ M. While a variety of labels can be used for the radiolabeled glucose employed in this invention, it is a requirement that such glucose be radiolabeled in such a manner that, upon production of glycogen, detectable radiolabeled glycogen is produced. Thus, it is preferable to use 1-¹⁴C glucose in the method of this invention.

Insulin is commercially available and can be obtained from various commercial sources such as Elanco Products Co., Indianapolis, Indiana 46285. Radiolabeled glucose is also commercially available from various suppliers such as Amersham Corp., 2636 South Clearbrook, Arlington Heights, Illinois 60065 and New England Nuclear, P.O. Box 80024, Wilmington, Delaware 19880.

Following completion of the incubation period, generally about 1 hour, in the presence of radiolabeled glucose, the muscle pieces are removed and assayed for radiolabeled glycogen as described below. The muscle pieces are placed in 1.0 ml of 1 N NAOH and maintained at about 60°C for about 1 hour. Then, 0.1 ml of a 100 mg/ml solution of glycogen is added. The glycogen is precipitated by adding 2.5 ml of ice cold 100% ethanol and storing ovemight at -20°C. The resulting precipitated glycogen is collected by centrifugation for 5 mins. at 3000 rpm in a table top centrifuge. The supematant is removed and the pellet is washed three times with ice cold 60% ethanol. After the last wash, the pellet is dissolved in 1.0 ml of water and counted in a liquid scintillation counter using standard methods and equipment.

Of course, the method described above can be applied mutatis mutandis using different volumes and vessels as well as muscle cells instead of muscle pieces. One skilled in the art, enabled by the disclosure herein, will readily appreciate such variations and make the appropriate changes such that the measure of glycogen can be calculated for such variations. All appropriate measurements of glycogen are within the scope of this invention.

A compound which is capable of inhibiting the ability of amylin to reduce insulin stimulated glycogen synthesis is identified as a compound which inhibits the ability of a polypeptide or a derivative thereof according to this invention to reduce insulin stimulated glycogen synthesis in muscle or muscle cells. The identification is effected by comparing the level of glycogen, for example ¹⁴C cpm, present in muscle or muscle cells when the compound is present with the level when the compound is absent. For those compounds which result in a higher level of glycogen produced, for example, a larger number of ¹⁴C cpm, then such compounds inhibit the ability of amylin to reduce insulin stimulated glycogen in muscle or muscle cells.

Pharmaceutically-acceptable salts of the polypeptides and derivatives thereof according to this invention are prepared according to standard methods well known to those skilled in art.

Pharmaceutical compositions comprising a polypeptide or a derivative thereof according to this invention can be prepared according to methods well known to those skilled in the art. For example, the polypeptide or derivative thereof can be combined with a pharmaceutically acceptable diluent or carrier. When the polypeptide or derivative thereof according to this invention are to be administered intravenously, intramuscularly, intraperitonealy or subcutaneously, appropriate sterile diluent is employed as is well known in the art. Such pharmaceutical compositions will comprise a sufficient amount of the polypeptide or derivative thereof so that an appropriate dosage, as hereinbelow described, can be administered over an appropriate period of time.

Dosages effective in suppressing insulin action in a mammal in need thereof are those which will result in a blood plasma concentration from about 10⁻¹⁰M to about 10⁻⁷M when a polypeptide or a derivative thereof according to this invention is administered intravenously, intramuscularly, intraperitonealy or subcutaneously. A preferred dosage range is one which will result in a blood plasma concentration of about 10⁻⁹M to about 10⁻⁸M. It is to be appreciated, however, that dosages outside of that range are possible and are also within the scope of this invention. The appropriate dosage can and will be determined by the prescribing physician and will be a result of the severity of the condition being treated as well as the response achieved with the polypeptide or derivative being administered and the age, weight, sex and medical history of the patient.

The following examples are illustrative of the invention and are not to be construed as limiting the scope of the invention in any way.

For those examples which follow wherein a polypeptide of this invention is prepared, the following conditions and materials were employed unless specifically noted otherwise. Synthesis of the polypeptides was performed on an Applied Biosystems (ABI) 430A peptide synthesizer (Applied Biosystems, Foster City, CA). Standard tBOC chemistry protocols were used as provided by ABI (Version 1.4 for N-methylpyrrolidone hydroxybenzotriazole chemistry). The protocols used hydroxybenzotriazole active ester coupling and acetic anhydride capping of unreacted free amino termini after each coupling. The resin samples were taken automatically at the end of each coupling and quantitative ninhydrin assay was used to monitor coupling efficiencies according to standard methods well known to those skilled in the art.

All tBOC amino acids were purchased from Applied Biosystems (Foster City, CA) in pre-loaded cartridges. The following amino acid side chain protection was used: p-chlorocarbobenzoxy for Lys, p-toluenesulfonyl for Arg, 2-bromocarbobenzoxy for Tyr, N-(π)-benzyloxymethyl for His, O-benzyl for Ser and Thr, S-acetomidomethyl (ACM) or S-4-methylbenzyl for Cys. All solid phase synthesis solvents and reagents were obtained from Applied Biosystems (Foster City, Ca.) except t-BOC Cys (ACM), which was obtained from Keystone Biotech (Philadelphia, PA).

The C-terminal amide polypeptides were assembled on benzhydryl amine resin (0.77 g, 0.65 mmol/g). Removal of the ultimate t-BOC protection was performed on the synthesizer at the end of the synthesis.

To remove the polypeptide from the resin, the polypeptide resin (500 mg) was suspended in 1 ml p-cresol and chilled to -78°C in a 40 ml Kel.F® reaction vessel attached in-line to a hydrofluoric acid (HF) apparatus (Peninsula Labs, Belmont, Ca.). The total volume was adjusted to 12 ml with condensed HF. After 1 hr. stirring at 0 to 5°C, the HF was removed in vacuo. The resulting residue was slurried in 3 ml trifluoroacetic acid and the solids were removed by filtration. The filtrate was precipitated into 40 ml diethyl ether. The precipitate was filtered, washed with fresh ether, and dried in vacuo.

To form the disulfide bond between Cys₂ and Cys₇, 100 mg (26µmol) crude polypeptide, prepared as described above, was dissolved in 6 M guanidine hydrochloride, 50 mM tris pH 8.5 and was treated with 31.6 mg (125 µmol) iodine dissolved in 15.6 ml acetic acid. After 2 hours reaction time, the solution was transferred to a 1000 Dalton cut-off Spectropor 6 (Spectrum, Los Angeles, Ca.) dialysis bag and dialyzed versus 33 mM sodium thiosulfite. When the iodine color faded from the diazylate, the thiosulfite dialysis buffer was replaced with distilled water; dialyzed versus distilled water (4 x 2 liter) with the final dialysis being allowed to run ovemight at 4°C. The diazylate was purified in two equal aliquots by preparative reverse phase HPLC on a Waters Delta Prep 3000 using a 20 x 250 mm Vydac C-18 Protein/Peptide column and a gradient elution system of 20% B, 80% A to 50% B, 50% A over 30 minutes (A is 5% acetonitrile/water/0.1% trifluoroacetic acid, B is 100% acetonitrile). Analytical data on the purified samples were obtained using an Plasma Desorption Mass Spectrometer (PDMS), Electrospray Mass Spectrometer (ES-MS), Fast Atom Bombardment Mass Spectrometer (FAB-MS) and amino acid analysis (AAA).

Analytical HPLC was performed using a 3.9 x 300 mm Waters µ-bondapack C-18 reverse phase column with a gradient of 20%B, 80% A to 50% B, 50% A over 30 minutes (A is 5% acetonitrile/water/0.1% trifluoroacetic acid, B is 100% acetonitrile).

Polypeptides synthesized using 4-methylbenzyl protection of the Cys thiol moieties were oxidized using iodine/acetic acid according to standard methods well known to those skilled in the art and subjected to HPLC purification as above.

### EXAMPLE 1

H₂N-R-CONH₂

wherein R is SEQUENCE NO:3, Xaa is Tyr and there is a disulfide bond between Cys₂ and Cys₇
Synthesis was performed as described above on 0.5 mmol scale using S-4-methylbenzyl protection of the Cys residues to afford 1.84 gm of resin bound polypeptide. The entirety of the resin was deprotected as described above affording 828 mg of crude polypeptide. HPLC purification of 7 mg of the crude polypeptide afforded 2.0 mg of polypeptide shown by ES-MS analysis to exhibit a mass of 3905.1 Da (expected 3905.06 Da). Disulfide bond formation was accomplished as described above on 50 mg of crude reduced peptide affording, after dialysis and HPLC purification, 1.7 mg of the title polypeptide. ES-MS analysis afforded a mass of 3903.3 Da (expected 3903.04 Da). Amino acid analysis exhibited the expected composition.

### EXAMPLE 2

H₂N-R-CONH₂

wherein R is SEQUENCE ID NO:3, Xaa is mono-l¹²⁴Tyr or bis-l¹²⁴ Tyr and there is a disulfide bond between Cys₂ Cys₇

To a solution of 27 µg (1.8 x 10⁻⁷ mol) of Nal¹²⁴ in 10 µl of 10 mM Tris pH 7.2 buffer was added 50µg of chloramine-T in 15µl of 10 mM Tris pH 7.2 buffer. After vortexing the solution for 15 seconds, a solution of 700 µg of the polypeptide produced in Example 1, above, in 100 µl of 10mM Tris pH 7.2 buffer was added and the iodination mixture was vortexed at ambient temperature for 5 min. The reaction was cooled to 0°C and subjected to preparative reverse phase analytical HPLC as described above. Two major fractions were collected. The mono-iodo Tyr containing polypeptide eluted at 13.5 minutes and exhibited a mass of 4028.6 Da by ES-MS analysis (expected 4028.46 Da). Amino acid analysis was consistent with the expected peptide composition and indicated a total yield for the mono-iodo Tyr containing title polypeptide of 33 µg. The bis-iodo Tyr containing polypeptide eluted at 14.0 minutes and exhibited a mass of 4154.6 Da by ES-MS (expected 4155.36 Da). Amino acid analysis was consistent with the expected peptide content and suggested a total yield for the bis-iodo Tyr containing title polypeptide of 67 µg.

### EXAMPLE 3

H₂N-R-CONH₂

wherein R is SEQUENCE ID NO:3, Xaa is mono-l¹²⁵ Tyr or bis-l¹²⁵Tyr and there is a disulfide bond between Cys₂ and Cys₇
To a solution of 0.29 µg/µl of the polypeptide prepared according to Example 1 in 60% acetonitrile/water 0.1%TFA, was added 80 µl 100 mM sodium phosphate buffer pH 7.4, 15 µl of solution containing 0.47 µg (5mCi) sodium iodide (l¹²⁵), 15 µl 15 mM hydrogen peroxide, and 32µl lactoperoxidase solution (1.7 µg/µl). The reaction mixture was vortexed for 1 hr. at room temperature. The mixture was then diluted with 200 µl of 1µg/µl bovine serum albumin (BSA) in 6 M guanidine-HCL, 50 mM Tris pH 6.1 and immediately loaded onto a Water's µbondapak C-18, 3.9 x 300 mm HPLC column. Both the mono-iodo and bis-iodo labeled polypeptides were collected as described in Example 2, above. A 5µl aliquot of a solution of 2 µg/µl BSA in water was added to each fraction. Co-injection of the bis-l¹²⁴ Tyr polypeptide
prepared according to Example 2 with the bis-l¹²⁵ Tyr polypeptide according to this Example were shown to exhibit identical retention times as evidenced by UV detection of the bis-l¹²⁴ Tyr and radio-detection of the bis-l¹²⁵ Tyr polypeptides by analytical HPLC as described above.

### EXAMPLE 4

H₂N-R-CONH₂

wherein R is SEQUENCE NO:4, Xaa is Tyr and there is a disulfide bond between Cys₂ and Cys₇
Synthesis was performed as described above on a 0.5 mmol scale using S-4-methylbenzyl protection of the Cys residues to afford 2.57 gm of resin polypeptide. A 600 mg portion of the resin was deprotected, as described above, affording 180 mg of crude polypeptide. HPLC purification of 7 mg of the crude polypeptide afforded 2.0 mg of polypeptide shown by PDMS analysis to exhibit a mass of 3922.5 Da (expected 3922.2 Da). Disulfide bond formation was accomplished as described above on 40 mg of crude polypeptide affording, after dialysis and HPLC purification, 4.0 mg of the title polypeptide. PDMS analysis afforded a mass of 3921.2 Da (expected 3920.2 Da). ES-MS analysis afforded a mass of 3919.9 Da (expected 3920.2 Da). Amino acid analysis exhibited the expected composition.

### EXAMPLE 5

H₂N-R-CONH₂

wherein R is SEQUENCE ID NO:4, Xaa is mono-l¹²⁴ Tyr or bis-l¹²⁴ Tyr and there is a disulfide bond between Cys₂ and Cys₇

Employing the procedure described in Example 2 with the polypeptide produced according to Example 4 afforded the title polypeptides. PDMS analysis of the mono-iodo Tyr containing polypeptide afforded a mass of 4046.8 Da ( expected 4046.08 Da). PDMS analysis of the bis-iodo Tyr containing polypeptide afforded a mass of 4173.5 Da (expected 4171.98 Da). Amino acid analysis of the polypeptides was consistent with the expected amino acid composition.

### EXAMPLE 6

H₂N-R-CONH₂

wherein R is SEQUENCE NO:4, Xaa is mono-l¹²⁵ Tyr or bis-l¹²⁵ Tyr and there is a disulfide bond between Cys₂ and Cys₇

Employing the procedure described in Example 3 with the polypeptide produced according to Example 4 afforded the title polypeptides.

## Claims

1. A polypeptide comprising the primary sequence
H₂N-R-CONH₂
wherein R is an amino acid sequence selected from the group consisting of and or a derivative thereof having insulin action suppressing activity, and the pharmaceutically acceptable salts thereof wherein Xaa is Tyr, mono-iodinated Tyr or bis-iodinated Tyr.

2. A polypeptide or a pharmaceutically acceptable salt thereof according to claim 1 wherein R is

3. A polypeptide according to claim 2 wherein there is a disulfide bond between Cys₂ and Cys₇.

4. A polypeptide or a pharmaceutically-acceptable salt thereof according to claim 3 wherein Xaa is Tyr.

5. A polypeptide or a pharmaceutically-acceptable salt thereof according to claim 3 wherein Xaa is mono-iodinated Tyr.

6. A polypeptide or a pharmaceutically-acceptable salt thereof according to claim 5 wherein the mono-iodinated Tyr is mono-l¹²⁵ Tyr.

7. A polypeptide or a pharmaceutically acceptable salt thereof according to claim 3 wherein Xaa is bis-iodinated Tyr.

8. A polypeptide or a pharmaceutically acceptable salt thereof according to claim 7 wherein the bis-iodinated Tyr is bis-l¹²⁵ Tyr.

9. A pharmaceutical composition comprising a polypeptide according to claim 1 and a pharmaceutically-acceptable diluent or carrier.

10. A method in vitro for suppressing insulin action in muscle or muscle cells which comprises incubating the muscle or muscle cells in the presence of an insulin action suppressing amount of a polypeptide according to any one of claims 1 to 8.

11. A method for suppressing insulin action in muscle or muscle cells according to claim 10 wherein the muscle is rat soleus muscle.

12. A method for assaying the ability of a compound to inhibit the suppression of insulin action in muscle or muscle cells caused by amylin which method comprises:
(a) incubating muscle or muscle cells in the presence of insulin, a polypeptide or derivative thereof according to any one of claims 1 to 8, the compound and glucose;
(b) measuring the amount of glycogen produced by the incubated muscle or muscle cells of step (a); and
(c) comparing the amount measured in step (b) to the amount measured according to step (b) when muscle or muscle cells are incubated in the presence of insulin, the polypeptide or derivative thereof and glucose.

13. A method according to claim 12 wherein radiolabeled glucose is used and radiolabeled glycogen is measured.

14. A method according to claim 12 wherein the muscle is rat soleus muscle.

15. A method according to claim 14 wherein the insulin is present at about 10⁻¹⁰ M to about 10⁻⁷ M.

16. A method according to claim 15 wherein the polypeptide is present at about 10⁻⁸ M to about 10⁻⁷ M.

17. A method according to claim 13 wherein the muscle or muscle cells are preincubated in the presence of insulin, the polypeptide or derivative thereof, the compound and glucose prior to step (a).

18. Use of a polypeptide according to any one of claims 1 to 8 for making a medicament for suppressing insulin action.

## Patentansprüche

1. Polypeptid mit der primären Sequenz H₂N-R-CONH₂, worin R eine Aminosäuresequenz ist, ausgewählt aus der Gruppe bestehend aus oder ein Derivat davon mit einer die Insulinwirkung unterdrückenden Akivität und die pharmazeutisch annehmbaren Salze davon, worin Xaa Tyr, monoiodiertes Tyr oder bisiodiertes Tyr ist.

2. Polypeptid oder pharmazeutisch annehmbares Salz davon gemäß Anspruch 1, worin R ist.

3. Polypeptid nach Anspruch 2, worin zwischen Cys₂ und Cys₇ eine Disulfidbindung besteht.

4. Polypeptid oder pharmazeutisch annehmbares Salz davon nach Anspruch 3, worin Xaa Tyr ist.

5. Polypeptid oder pharmazeutisch annehmbares Salz davon nach Anspruch 3, worin Xaa monoiodiertes Tyr ist.

6. Polypeptid oder pharmazeutisch annehmbares Salz davon nach Anspruch 5, worin das monoiodierte Tyr Mono-I¹²⁵-Tyr ist.

7. Polypeptid oder pharmazeutisch annehmbares Salz davon nach Anspruch 3, worin Xaa bisiodiertes Tyr ist.

8. Polypeptid oder pharmazeutisch annehmbares Salz davon nach Anspruch 7, worin das bisiodierte Tyr Bis-I¹²⁵-Tyr ist.

9. Pharmazeutische Zusammensetzung enthaltend ein Polypeptid nach Anspruch 1 und ein pharmazeutisch annehmbares Verdünnungsmittel oder einen pharmazeutisch annehmbaren Träger.

10. Methode, um die Insulinwirkung in Muskeln oder Muskelzellen in vitrc zu unterdrücken, das umfaßt, daß man den Muskel oder die Muskelzellen in Gegenwart einer die Insulinwirkung unterdrückenden Menge eines Polypeptids nach einem der Ansprüche 1 bis 8 inkubiert.

11. Verfahren zur Unterdrückung der Insulinwirkung in Muskeln oder Muskelzellen nach Anspruch 10, worin der Muskel Soleusmuskel von der Ratte ist.

12. Verfahren, um die Fähigkeit einer Verbindung, die Unterdrückung der Insulinwirkung in Muskeln oder Muskelzellen, die durch Amylin verursacht wird, zu hemmen, zu untersuchen, wobei das Verfahren umfaßt, daß man:
(a) Muskel oder Muskelzellen in Gegenwart von Insulin, einem Polypeptid nach einem der Ansprüche 1 bis 8 oder einem Derivat davon, der Verbindung und Glucose inkubiert;
(b) die Menge an in Stufe (a) von dem inkubierten Muskel oder den inkubierten Muskelzellen erzeugten Glycogen mißt
(c) die in Stufe (b) gemessene Menge mit der in Stufe (b) gemessenen Menge, wenn Muskel oder Muskelzellen in Gegenwart von Insulin, dem Polypeptid oder Derivat davon und Glucose inkubiert wurden, verglichen wird.

13. Verfahren nach Anspruch 12, worin radioaktiv markierte Glucose verwendet wird und radioaktiv markiertes Glycogen gemessen wird.

14. Verfahren nach Anspruch 12, worin der Muskel Soleusmuskel von der Ratte ist.

15. Verfahren nach Anspruch 14, worin Insulin in einem Anteil von etwa 10⁻¹⁰ M bis etwa 10⁻⁷ M vorhanden ist.

16. Verfahren nach Anspruch 15, worin das olypeptid in einem Anteil von etwa 10⁻⁸ bis etwa 10⁻⁷ M vorhanden ist.

17. Verfahren nach Anspruch 13, worin der Muskel oder die Muskelzellen in Gegenwart von Insulin, dem Polypeptid oder Derivat davon, der Verbindung und Glucose vor der Stufe (a) vorinkubiert werden.

18. Verwendung eines Polypeptids nach einem der Ansprüche 1 bis 8 zur Herstellung eines Arzneimittels zur Unterdrükkung der Insulinwirkung.

## Revendications

1. Polypeptide comprenant la séquence fondamentale:
H₂N-R-CONH₂
dans laquelle R est une séquence d'acides aminés choisie dans le groupe consistant en: et: ou dérivé de celui-ci ayant une activité de suppression de l'action de l'insuline, et sels acceptables du point de vue pharmaceutique de ceux-ci, où Xaa est Tyr, Tyr mono-iodé ou Tyr bi-iodé.

2. Polypeptide ou sel de celui-ci acceptable du point de vue pharmaceutique suivant la revendication 1, dans lequel R est:

3. Polypeptide suivant la revendication 2, dans lequel il existe une liaison di-sulfure entre Cys₂ et Cys₇.

4. Polypeptide ou sel de celui-ci acceptable du point de vue pharmaceutique suivant la revendication 3, dans lequel Xaa est Tyr.

5. Polypeptide ou sel de celui-ci acceptable du point de vue pharmaceutique suivant la revendication 3, dans lequel Xaa est Tyr mono-iodé.

6. Polypeptide ou sel de celui-ci acceptable du point de vue pharmaceutique suivant la revendication 5, dans lequel le Tyr mono-iodé est mono-I¹²⁵ Tyr.

7. Polypeptide ou sel de celui-ci acceptable du point de vue pharmaceutique suivant la revendication 3, dans lequel Xaa est Tyr bi-iodé.

8. Polypeptide ou sel de celui-ci acceptable du point de vue pharmaceutique suivant la revendication 7, dans lequel le Tyr bi-iodé est le bi-l¹²⁵ Tyr.

9. Composition pharmaceutique comprenant un polypeptide suivant la revendication 1 et un diluant ou support acceptable du point de vue pharmaceutique.

10. Procédé *in vitro* pour supprimer l'action de l'insuline dans un muscle ou des cellules musculaires qui comprend l'incubation du muscle ou des cellules musculaires en présence d'une quantité supprimant l'action de l'insuline d'un polypeptide suivant l'une quelconque des revendications 1 à 8.

11. Procédé pour supprimer l'action de l'insuline dans un muscle ou des cellules musculaires suivant la revendication 10, dans lequel le muscle est un muscle soléaire de rat.

12. Procédé pour déterminer l'aptitude d'un composé à inhiber la suppression de l'action de l'insuline dans un muscle ou des cellules musculaires provoquée par l'amyline, lequel procédé comprend:
(a) l'incubation d'un muscle ou de cellules musculaires en présence d'insuline, d'un polypeptide ou d'un dérivé de celui-ci suivant l'une quelconque des revendications 1 à 8, du composé et de glucose;
(b) la mesure de la quantité de glycogène produit par le muscle ou les cellules musculaires incubés de l'étape (a); et
(c) la comparaison de la quantité mesurée dans l'étape (b) à la quantité mesurée suivant l'étape (b) lorsque le muscle ou les cellules musculaires sont incubés en présence d'insuline. du polypeptide ou de son dérivé et de glucose.

13. Procédé suivant la revendication 12, dans lequel du glucose radiomarqué est utilisé et du glycogène radiomarqué est dosé.

14. Procédé suivant la revendication 12, dans lequel le muscle est un muscle soléaire de rat.

15. Procédé suivant la revendication 14, dans lequel l'insuline est présente à raison d'environ 10⁻¹⁰ M à environ 10⁻⁷ M.

16. Procédé suivant la revendication 15, dans lequel le polypeptide est présent à raison d'environ 10⁻⁸ M à environ 10⁻⁷ M.

17. Procédé suivant la revendication 13, dans lequel le muscle ou les cellules musculaires sont préincubés en présence d'insuline, du polypeptide ou d'un dérivé de celui-ci, du composé et de glucose avant l'étape (a).

18. Utilisation d'un polypeptide suivant l'une quelconque des revendications 1 à 8 pour la fabrication d'un médicament pour supprimer l'action de l'insuline.
